# EUROPEAN PATENT APPLICATION

(11) **EP 4 736 857 A1**
(43) Date of publication of application: **06.05.2026**
(21) Application number: 25212418.5
(22) Date of filing: 30.10.2025
(51) Int. Cl.: A61K 31/593, A61K 31/592, A61K 36/282, A61K 36/31, A61K 36/324, A61K 45/06, A61P 19/10

(54) **MIXTURES AND NUTRACEUTICAL COMPOSITIONS COMPRISING ARTEMISIA EXTRACT, ERUCA EXTRACT, BOSWELLIA EXTRACT AND VITAMIN D, AND THEIR USE IN THE PREVENTION AND TREATMENT OF OSTEOPOROSIS**

(30) Priority: 30.10.2024 IT 202400024249
(71) Applicant: Nutra Futura S.r.l., 20155 Milano (IT)
(72) Inventor: UBERTI, Francesca, 28838 Stresa (Verbano-Cusio-Ossola) (IT); FAÈ, Annalisa, 35038 Torreglia (Padova) (IT)
(74) Representative: Delbarba, Andrea

(57) **Abstract**

The present invention relates to a mixture, food, nutraceutical and/or pharmaceutical compositions comprising it and their use in prevention and/or slowdown and/or treatment of osteoporosis.

## Description

The present invention relates to a mixture and a composition comprising an Artemisia extract, a Boswellia extract, an Eruca extract and at least one vitamin D, and their use in prevention and/or slowdown and/or treatment of osteoporosis. The invention also relates to a non-medical use of the mixture or the composition to maintain bone homoeostasis in an individual.

### STATE OF THE ART

Osteoporosis is a systemic disease of the skeletal system, characterised by a low mineral density and by the deterioration of the microarchitecture of the bone tissue, which becomes more fragile and more exposed to a risk of spontaneous fractures or fractures caused by minor traumas. Osteoporosis is divided into two forms: primary, which includes the postmenopausal and senile varieties, and secondary, which is due to different pathologies and to the taking of certain drugs over the medium/long-term. In more detail, in the case of osteoporosis, the physiological process of remodelling, in which the old and damaged skeletal tissue is removed by osteoclasts and new bone is reformed by osteoblasts, is altered. In fact, with age, the activity of the osteoclasts tends to be greater than the activity of the osteoblasts, with a consequent loss in bone mass.

As for most diseases, osteoporosis can be prevented by adopting a healthy lifestyle, with regular exposure to the sun, physical exercise, following a diet that provides for a sufficient intake of foods with a high calcium content, and avoiding abuse of alcohol and smoking of cigarettes. However, these precautions are often not sufficient to prevent the onset of the disease.

In fact, genetics and heredity play a leading role in the development of osteoporosis and in susceptibility to fractures.

In this regard, in fact, the results of a genome-wide meta-analysis study published in Nature Genetics discovered that variants of 56 genome regions influence the bone density (BMD, *Bone mineral density*) of an individual and 14 of these variants are associated with an increase in the risk of fracture (Estrada K., Styrkarsdottir U., Evangelou E. et al. Genome-wide meta-analysis identifies 56 bone mineral density loci and reveals 14 loci associated with risk of fracture. Nat Genet 44, 491-501 (2012). Therefore, in these cases, it is necessary to act on slowing down the onset of osteoporosis.

It is therefore necessary to provide a product (mixture and/or composition) capable of preventing and/or slowing down the onset of osteoporosis.

It is also useful to provide a food, nutraceutical and/or pharmaceutical composition for use in the treatment of osteoporosis, which contains natural ingredients and therefore does not result in the known side effects of the drugs commonly used for the treatment of osteoporosis. Anti-osteoporosis drugs act on skeletal remodelling by restoring the balance between bone resorption and new bone formation. Most of the drugs used as the first line (bisphosphonates, denosumab, selective estrogen receptor modulators - SERM) act by inhibiting the function of the osteoclasts and therefore bone resorption. However, there are known main adverse effects common to most of the active ingredients in the class of bisphosphonates (nausea, abdominal pain, diarrhoea, and esophagitis), denosumab (hypercholesterolemia, bladder infections, hypocalcemia, serious infections, including skin infections, dermatitis, eczema and skin rashes, osteonecrosis of the jaw/lower jaw) and SERM (hot flushes, muscle spasms, increase in hematic levels of triglycerides and/or hepatic enzymes and sleepiness, among the most common).

Considering the strong side effects caused by the drugs commonly used for the treatment of osteoporosis, there is therefore a need to provide products (mixtures and/or compositions) that do not result in heavy adverse effects in individuals who are often elderly and who, in any case, already have, in certain cases, one or more fractures.

The technical problem that the present invention addresses and solves is thus to provide a product (mixture and/or composition) that does not cause side effects and that is capable of preventing and/or slowing down and/or treating osteoporosis.

Furthermore, it is necessary to provide a mixture and/or a composition which are useful for that purpose, easy to prepare and free of side effects, and which do not require the use of costly machinery and processes.

### SUMMARY OF THE INVENTION

A first aspect of the present invention relates to a mixture comprising or, alternatively, consisting of:
i) an Artemisia extract;
ii) a *Boswellia* extract;
iii) an Eruca extract; and
iv) at least one vitamin D.

Preferably, said Artemisia extract comprises artemisinin.

In one embodiment, the *Artemisia* extract is an *Artemisia annua* extract, and/or said *Boswellia* extract is a *Boswellia serrata* extract, and/or said *Eruca* extract is an *Eruca sativa Mill.* extract.

Preferably, the *Artemisia* extract has an artemisinin titre comprised from 80% to 100% by weight; more preferably, comprised from 85% to 99.9% by weight; even more preferably, comprised from 90% to 99.7% by weight, with respect to the total weight of the extract.

Preferably, the *Boswellia* extract comprises at least one keto-boswellic acid, preferably the keto-boswellic acid is 3-acetyl-11-keto-β-boswellic acid (AKBA).

Preferably, said at least one keto-boswellic acid is present in said *Boswellia* extract in a concentration comprised from 20% to 70% by weight; more preferably, comprised from 25% to 60%; even more preferably, comprised from 30% to 50% by weight, with respect to the total weight of the extract.

In one embodiment, the *Eruca* extract comprises icariins, preferably, the icariins are present in said *Eruca* extract in a concentration comprised from 5% to 50% by weight, preferably, comprised from 10% to 40% by weight; more preferably, comprised from 15% to 25% by weight, with respect to the total weight of the extract.

Preferably, the at least one vitamin D is chosen from the group comprising, or alternatively consisting of, vitamin D1, vitamin D2, vitamin D3, vitamin D4,and vitamin D5, or mixtures thereof; preferably, said vitamin D is chosen from among vitamin D2 and vitamin D3, or mixtures thereof; more preferably, said vitamin D is vitamin D3.

A second aspect relates to a composition comprising the mixture described above and optionally at least one acceptable pharmaceutical or food grade additive and/or excipient.

Preferably, the composition is formulated in the form of a capsule, a tablet, a sachet, a stick, an orosoluble stick, an oral gel or granules.

A third aspect of the present invention relates to the mixture or the composition described above for use as a medicament.

A fourth aspect of the present invention relates to the mixture or the composition described above for use in the treatment or in the prevention of osteoporosis.

A fifth aspect of the present invention relates to the non-medical or nutraceutical use of the mixture or the composition described above to maintain the homoeostasis of bone remodelling by preserving the balance between resorption and formation even during an osteoporotic condition, and/or to maintain bone mineral density and/or for maintaining the bone microarchitecture and/or for reduction of the risk of fractures caused by bone fragility in an individual.

### DESCRIPTION OF THE FIGURES

Figure 1: cell viability with respect to the control following treatment with different concentrations of the individual active ingredients. The data are expressed as the mean ± DS (%) of 5 independent experiments, each performed in triplicate and expressed in (%). All the data p<0.05.
Figure 2: cell viability following treatment with the individual substances and the mixture (MIX) of the invention with respect to the control, at different times. The data are expressed as the mean ± DS (%) of 5 independent experiments, each performed in triplicate and expressed in (%).
Figure 3: Reactive oxygen species (ROS) production after treatment with the individual components and the mixture (MIX). The data are expressed as the mean ± DS (%) of 5 independent experiments, each performed in triplicate and expressed in (%).
Figure 4: measurement of transepithelial resistance for the individual compounds and for the mixture (MIX). The data are expressed as the mean ± DS (%) of 5 independent experiments, each performed in triplicate and expressed in (%).
Figure 5: analysis of the tight junctions Claudin 4, Occludin and ZO-1. The data are expressed as the mean ± DS (%) of 5 independent experiments, each performed in triplicate and expressed in (%).
   * p <0.05 v. control; # p < 0.05 v. single agents
Figure 6: analysis of the activity of alkaline phosphatase (differentiation marker) and % of mineralisation with respect to the control, obtained by treating the MC3T3-E1 cells with the individual compounds, with the mixture (MIX) and with dexamethasone. The data are expressed as the mean ± DS (%) of 5 independent experiments, each performed in triplicate and expressed in (%).
   * p<0.05 v. control; # p<0.05 v. single agents; φ p<0.05 v. dexamethasone.
Figure 7: number of calcium deposits (visible after staining with *Alizarin red*) in the untreated control, in the sample treated with dexamethasone and in the sample treated with the mixture (indicated in Figure 7 as "study MIX"). The calcium deposits are indicated in the figure with arrows. The figures were obtained by means of microscope with Leica DMi1 camera (Leica, Germany). The photos were taken with an enlargement of 20x.
Figure 8: TRAP analysis (%) in a 3D co-culture with MC3T3-E1 cells. The data are expressed as the mean ± DS (%) of 5 independent experiments, each performed in triplicate and expressed in (%).
   * p<0.05 v. control; # p<0.05 v. single agents; φ p<0.05 v. dexamethasone.
   The right-hand panel of Figure 8 shows the bone resorption activity in the 3D co-culture of RAW 264.7 and MC3T3-E1 cells. The data are expressed as the mean ± DS (%) of 5 independent experiments, each performed in triplicate and expressed in (%).
   * p<0.05 v. control; # p<0.05 v. single agents; φ p<0.05 v. dexamethasone.

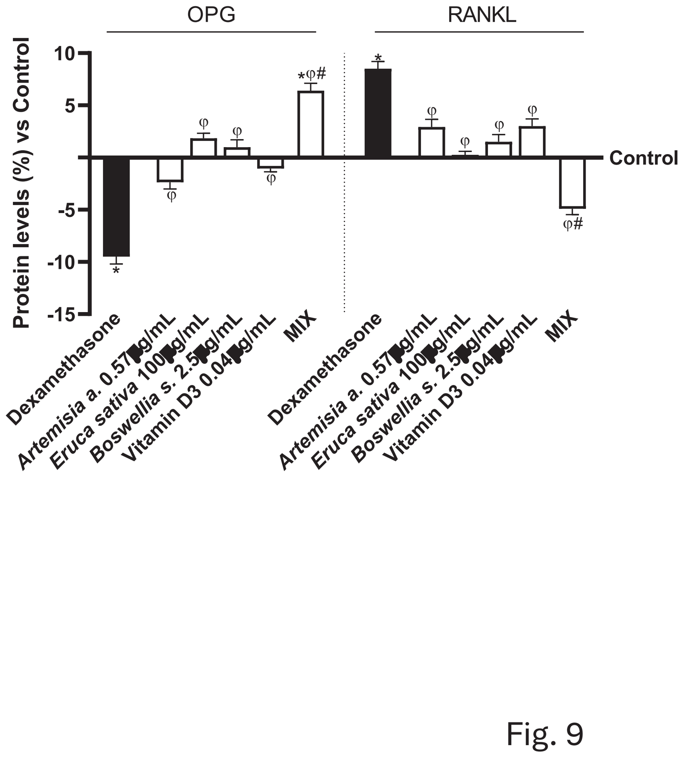

Figure 9: level of OPG and RANKL proteins found on the RAW 264.7 co-culture with MC3T3-E1 cells after treatment with the single compounds and with the mixture (MIX). The data are expressed as the mean ± DS (%) of 5 independent experiments, each performed in triplicate and expressed in (%).
   * p<0.05 v. control; # p<0.05 v. single agents; φ p<0.05 v. dexamethasone.

### DETAILED DESCRIPTION OF THE INVENTION

**A first** aspect of the present invention relates to a mixture comprising or, alternatively, consisting of:
i) an *Artemisia* extract;
ii) a *Boswellia* extract;
iii) an *Eruca* extract; and
iv) at least one vitamin of the D group.

The data obtained with the mixture represent important experimental evidence and confirm, advantageously, that the mixture comprising the extracts is capable of: a) crossing the intestinal epithelium without altering the integrity thereof, b) improving the functions of the osteoblasts, c) playing a crucial role in mineralisation of the newly formed bone, increasing it during osteoblast differentiation, d) playing a crucial role in calcification of the newly formed bone, e) inhibiting the activity of the osteoclasts, reducing osteoclastogenesis and bone resorption, f) maintaining homoeostasis of bone remodelling by preserving the balance between resorption and formation even during an osteoporotic condition.

Preferably, the Artemisia extract comprises at least one artemisinin.

In one embodiment, said Artemisia extract is a dry extract of *Artemisia* obtained from *Artemisia annua* leaves.

Preferably, said Artemisia extract comprises an artemisinin titre preferably comprised from 85% to 99.9% by weight; even more preferably, comprised from 90% to 99.7% by weight, for example 99.6% by weight with respect to the total weight of the extract, measured by means of HPLC.

From a chemical viewpoint, the artemisinin is a sesquiterpene lactone endoperoxide with molecular formula C₁₅H₂₂O₅ and CAS number, for example, of 63968-64-9.

In the context of the present invention, *"Artemisia* extract" is understood to include all types of *Artemisia* extracts comprising artemisinin.

Preferably, said extract is a dry extract of *Artemisia,* more preferably a dry extract of *Artemisia annua.*

*Boswellia* is a plant genus belonging to the *Burseraceae* family, which comprises around 20 species.

In the context of the present invention, *Boswellia* extract is understood to include all types of *Boswellia* extracts comprising at least one keto-boswellic acid and/or a salt or derivative thereof.

Therefore, preferably, said *Boswellia* extract is an extract titred in keto-boswellic acid.

More preferably, said extract can be a dry extract of *Boswellia serrata.* Preferably, said *Boswellia* extract is a *Boswellia serrata* extract titred in keto-boswellic acid.

Therefore, the mixture according to the invention comprises ii) at least one keto-boswellic acid, preferably present in a phytocomplex, wherein said phytocomplex is present in a *Boswellia* extract, more preferably a *Boswellia serrata* extract.

In one embodiment, the Boswellia extract comprises at least one keto-boswellic acid.

Preferably, said *Boswellia* extract is a dry extract of *Boswellia serrata* obtained from the gum resin of *Boswellia serrata* through ethyl acetate extraction. The extraction ratio is preferably comprised from 50:1 to 5:1, preferably from 40:1 to 10:1, more preferably from 30:1 to 25:1, for example 20:1.

Preferably, the titre of the total organic acids present in the Boswellia extract is comprised from 50% to 90%, preferably from 60% to 80% by weight.

Said *Boswellia* extract, preferably a *Boswellia serrata* extract, has a keto-boswellic acid titre comprised from 20% to 70% by weight, preferably comprised from 25% to 60% by weight, more preferably comprised from 30% to 50% by weight, for example 30% by weight with respect to the total weight of the dry extract, measured by means of HPLC.

Preferably, said at least one keto-boswellic acid is a 3-acetyl-11-keto-β-boswellic acid (AKBA) , for example having CAS number: 67416-61-9, and/or acetyl-β-boswellic acid and/or acetyl-α-boswellic acid.

Preferably, the 3-acetyl-11-keto-β-boswellic acid (AKBA) is present in a concentration comprised between 5 and 15% by weight, preferably comprised between 8 and 12% by weight.

Preferably, the acetyl-β-boswellic acid is present in a concentration comprised between 10 and 30% by weight, preferably comprised between 15 and 25% by weight.

Preferably, the acetyl-α-boswellic acid is present in a concentration comprised between 2 and 15% by weight, preferably comprised between 3 and 8% by weight.

Preferably, the Eruca extract comprises icariins.

Icariins (for example, having CAS number: 489-32-7) are chemical compounds belonging to flavonoids and, in particular, from a chemical viewpoint, are 8-prenyl kaempferol 3,7-O-diglucoside derivatives. Preferably, said *Eruca* extract is an *Eruca sativa* extract, more preferably it is an *Eruca sativa Mill.* extract.

*Eruca sativa* is an annual herbaceous plant in the *Brassicaceae* family and is commonly known as garden rocket.

In the context of the present invention, *Eruca sativa* extract is understood to include all types of *Eruca sativa* extracts comprising icariins.

Preferably, said *Eruca sativa* extract, preferably an *Eruca sativa Mill.* extract, has an icariin titre preferably comprised from 5% to 50% by weight, more preferably comprised from 10% to 40% by weight, more preferably comprised from 15% to 25% by weight, for example 10.41% by weight with respect to the total weight of the dry extract, measured by means of HPLC.

Said dry extract of *Eruca sativa* can be used as a source of icariins.

In one embodiment, the Eruca extract is obtained from *Eruca sativa Mill.* leaves.

Preferably, the dry extract of *Eruca sativa Mill.* has an icariin titre comprised preferably from ≥ 10% to lower than or equal to 25% by weight, preferably comprised from 12% to 18%, for example 10.4% by weight with respect to the total weight of the dry extract, measured by means of HPLC. Preferably, said iv) at least one vitamin D is chosen from the group comprising or, alternatively, consisting of vitamin D1, vitamin D2, vitamin D3, vitamin D4, vitamin D5 or mixtures thereof.

Preferably, said vitamin D is chosen from among vitamin D2 and vitamin D3, or mixtures thereof.

More preferably, the vitamin D present in the mixture of the invention is vitamin D3.

An example of a commercial product comprising vitamin D3 can, without constraints or limitations, be, for example, the product with the name "Vitamin D3 Powder 100000IU/g Food Grade CWD" marketed by Xiamen Kingdomway Biotech. Co., Ltd.

A **second** aspect of the present invention relates to a composition comprising the mixture described above and at least one pharmaceutically acceptable excipient or vehicle.

Preferably, the composition comprises or, alternatively, consists of:
i) an Artemisia extract in a concentration comprised between 10 and 25% weight/weight of the composition, preferably comprised between 12 and 18% w/w,
ii) a Boswellia extract in a concentration comprised between 30 and 65% weight/weight of the composition, preferably comprised between 45 and 55% w/w;
iii) an Eruca extract in a concentration comprised between 20 and 45% weight/weight of the composition, preferably comprised between 25 and 40% w/w; and
iv) at least one vitamin D in a concentration comprised between 0.005 and 0.007% weight/weight of the composition, preferably comprised between 0.006 and 0.008 % w/w.

In one embodiment, a composition having a final weight of 700 mg comprises or consists of:
- dry extract from 50 to 500 mg, preferably from 80 to 200 mg, more preferably from 100 to 150 mg, even more preferably 100 mg of the Artemisia extract;
- from 100 mg to 800 mg, preferably from 200 mg to 600 mg, more preferably from 300 mg to 400 mg, even more preferably 350 mg of the Boswellia extract;
- from 100 to 800 mg, preferably from 150 to 600 mg, even more preferably from 200 to 400 mg, even more preferably 250 mg of the Eruca extract; and
- from 0.001 to 0.1 mg, preferably from 0.01 mg to 0.08 mg, for example 0.05 mg, of iv) vitamin D and/or derivatives thereof, preferably vitamin D3; and
at least one acceptable pharmaceutical or food grade additive and/or excipient.

Said composition is, for example, a pharmaceutical composition or a nutraceutical composition or a supplement composition or a food composition or a composition for medical devices, for example according to Reg. (EU) 745/2017, or a composition for foods for special medical purposes, for example according to Reg. (EU) 609/2013.

As excipients, it is possible to use organic or inorganic substances, or solid or liquid substances as conventionally used excipients and vehicles, provided they are edible, physiologically acceptable and compatible with all the other components of the composition.

A person skilled in the art is perfectly capable of selecting the most suitable vehicles and excipients for preparation of the composition. Non-limiting examples of suitable vehicles and excipients are described in Remington: The Science and Practice of Pharmacy, 21st Ed., Lippincott, Williams & Wilkins.

The types of food, nutraceutical and/or pharmaceutical additives used to prepare the compositions of the invention, the ratios of the additive contents relative to the active ingredients and the methods for preparing the food, nutraceutical and/or pharmaceutical composition can be appropriately selected by the person skilled in the art.

The composition of the invention is a pharmaceutical and/or nutraceutical composition for oral use, preferably a composition in a solid state, preferably formulated in dosage units. "Solid state" means that the composition can exist in the form of granules or powders. The granular or powder compositions are mixed with pharmaceutically acceptable additives and excipients to provide a final product such as, for example, a supplement product, a medical device or a pharmaceutical composition. The final product may be in pharmaceutical dosage units such as, for example, granules in a sachet, a stick, orosoluble stick, tablet, gel or capsule.

The tablets may have different shapes among those known in the field of pharmaceutical forms, such as, for example, a cylindrical or spheroidal shape. The tablets may be coated or film-coated with one or more coating or film layers capable of passing through the gastric barrier, according to known methods.

The gel capsules may consist of hard gelatine or soft gelatine or soft gel. Preferably, the oral composition of the invention is a solid or semi-solid (gel) composition as described above. However, if desired or necessary, the composition may be formulated in liquid form, for example by dissolution or suspension in water.

Preferably, the compositions of the present invention may be in the form of a capsule, a tablet, a sachet, a stick, an orosoluble stick, an oral gel or granules.

A **third** aspect of the present invention relates to the mixture or the composition described above for use as a medicament.

A **fourth** aspect of the present invention relates to the mixture or the composition described above for use in the treatment or in the prevention of osteoporosis.

The composition of the invention, preferably formulated in dosage units, may be administered once or several times a day, for example once or twice a day, preferably twice a day.

The applicant has found that the mixture of the invention and/or the pharmaceutical and/or nutraceutical compositions that contain it are useful for maintaining the homoeostasis of bone remodelling by preserving the balance between resorption and formation even during an osteoporotic condition.

The daily dosage depends on the patient's health status, weight, gender and age. In general, the compositions of the invention are well tolerated and can be administered once or several times a day, preferably twice a day, even over an extended period of time.

A **fifth** aspect of the present invention relates to the non-medical or nutraceutical use of the mixture or the composition described above to maintain the homoeostasis of bone remodelling by preserving the balance between resorption and formation even during an osteoporotic condition, and/or to maintain bone mineral density and/or for maintaining the bone microarchitecture and/or for reduction of the risk of fractures caused by bone fragility in an individual.

The present description relates to a method for treating osteoporosis that comprises administering, to an individual who needs it, the mixture and/or composition of the invention, preferably in a dosage effective for the purpose.

Advantageously, the mixture and/or the composition according to the invention can be administered on their own or, possibly, together with the drugs and/or dietary supplements commonly used for the treatment of osteoporosis.

Furthermore, the mixture of the invention and the pharmaceutical and/or nutraceutical compositions containing it, due to the presence of natural ingredients, do not have significant adverse effects, are free of side effects and have high tolerability, and can be administered to a broad category of individuals.

Representative embodiments of the compositions of the invention are given in the Experimental Section below, solely by way of illustration and in no way limiting.

### EXAMPLES

### Example 1

Composition in the form of a tablet or sachet or orosoluble stick or gel stick (1 dosage form) comprising:

**Table 1**

| **Components** | **Dosage form** |
|---|---|
| dry extract of *Artemisia annua* of which artemisinin at 99.62% | 100 mg |
| | 99.62 mg |
| dry extract of *Boswellia serrata* of which keto-boswellic acid 30% | 350 mg |
| | 105 mg |
| dry extract of *Eruca sativa Mill.* of which icariins at 10.41 % | 250 mg |
| | 26.025 mg |
| Vitamin D3 | 0.05 mg |

| | |
|---|---|
| together with food or pharmaceutical grade excipients and/or additives. | |

### Example 2

Composition in the form of a tablet or sachet or orosoluble stick or gel stick (1 dosage form) containing:

**Table 2**

| **Components** | **Dosage form** |
|---|---|
| dry extract of *Artemisia annua* of which artemisinin at 99% | 100 mg |
| | 99 mg |
| dry extract of *Boswellia serrata* of which keto-boswellic acid 40% | 350 mg |
| | 140 mg |
| dry extract of *Eruca sativa Mill.* of which icariins at 15 % | 250 mg |
| | 37.5 mg |
| Vitamin D3 | 0.05 mg |

| | |
|---|---|
| together with food or pharmaceutical grade excipients and/or additives. | |

### Example 3

Composition in the form of a tablet or sachet or orosoluble stick or gel stick (1 dosage form) containing:

**Table 3**

| **Components** | **Dosage form** |
|---|---|
| dry extract of *Artemisia annua* of which artemisinin at 99.5% | 100 mg |
| | 99.5 mg |
| dry extract of *Boswellia serrata* of which keto-boswellic acid 35% | 350 mg |
| | 122.5 mg |
| dry extract of *Eruca sativa Mill.* of which icariins at 10% | 250 mg |
| | 25 mg |
| Vitamin D3 | 0.025 mg |

| | |
|---|---|
| together with food or pharmaceutical grade excipients and/or additives. | |

### Example 4

Composition in the form of a tablet or sachet or orosoluble stick or gel stick (1 dosage form) containing:

**Table 4**

| **Components** | **Dosage form** |
|---|---|
| dry extract of *Artemisia annua* of which artemisinin at 99% | 100 mg |
| | 99 mg |
| dry extract of *Boswellia serrata* of which keto-boswellic acid 40% | 350 mg |
| | 140 mg |
| dry extract of *Eruca sativa Mill.* of which icariins at 15 % | 250 mg |
| | 37.5 mg |
| Vitamin D3 | 0.05 mg |

| | |
|---|---|
| together with food or pharmaceutical grade excipients and/or additives. | |

### Example 5

Composition in the form of a tablet or sachet or orosoluble stick or gel stick (1 dosage form) containing:

**Table 5**

| **Components** | **Dosage form** |
|---|---|
| dry extract of *Artemisia annua* of which artemisinin at 80% | 200 mg |
| | 160 mg |
| dry extract of *Boswellia serrata* of which keto-boswellic acid 35% | 300 mg |
| | 105 mg |
| dry extract of *Eruca sativa Mill.* of which icariins at 18 % | 200 mg |
| | 36 mg |
| Vitamin D3 | 0.08 mg |

| | |
|---|---|
| together with food or pharmaceutical grade excipients and/or additives. | |

### Example 6

Composition in the form of a tablet or sachet or orosoluble stick or gel stick (1 dosage form) containing:

**Table 6**

| **Components** | **Dosage form** |
|---|---|
| dry extract of *Artemisia annua* of which artemisinin at 90% | 150 mg |
| | 135 mg |
| dry extract of *Boswellia serrata* of which keto-boswellic acid 25% | 400 mg |
| | 100 mg |
| dry extract of *Eruca sativa Mill.* of which icariins at 20 % | 150 mg |
| | 30 mg |
| Vitamin D3 | 0.075 mg |

| | |
|---|---|
| together with food or pharmaceutical grade excipients and/or additives. | |

### In vitro study

### 1. Experimental protocol

An *in vitro* study was conducted with the objective of investigating the mechanism of action through which the components of the mixture exert their effect, tested on their own or in a mixture ("MIX" in the Figures), in slowing down osteoporosis.

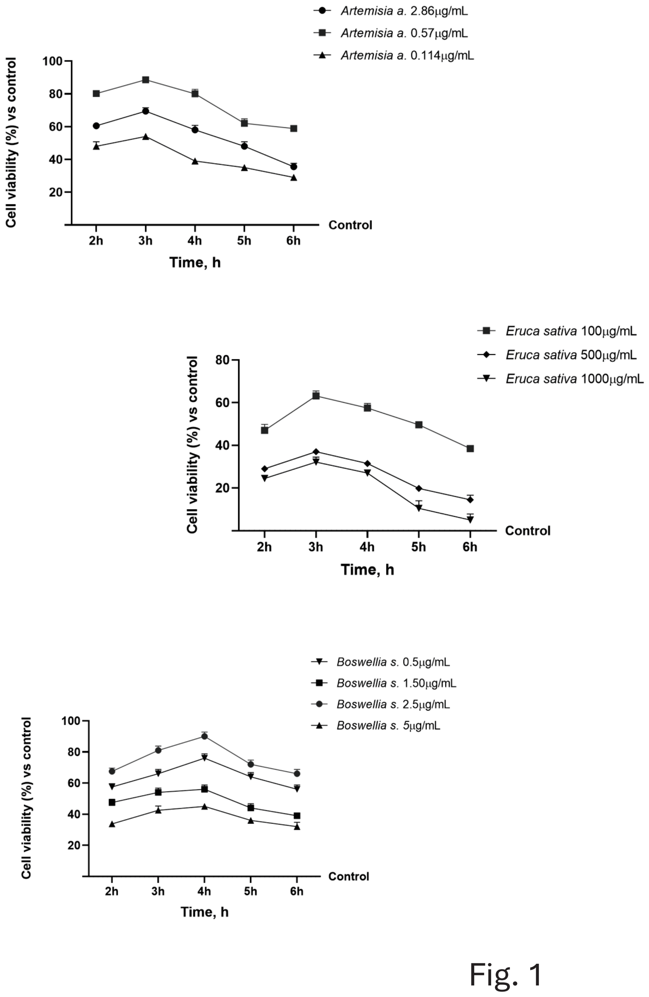

The components present in the mixture were tested in the different concentrations indicated here below, in order to measure the effect of the different concentrations of substance on cell viability of CaCo-2 cells compared to the control (represented by cells in baseline conditions not treated with the substances) (Figure 1).

In particular, the components were tested at the following concentrations:
1. extract of *Artemisia annua* at 99.62% tested in concentrations from 0.114 to 2.86 µg/mL
2. extract of *Boswellia Serrata* at 30% tested in concentrations from 0.5 to 5 µg/mL
3. extract of *Eruca sativa* at 10.41% tested in concentrations from 100 to 1000 µg/mL.

The mixture (MIX) then tested in the experiments indicated here below comprises:
- 0.57 µg/mL *Artemisia annua* at 99.62%;
- 2.5 µg/mL *Boswellia Serrata* at 30%;
- 100 µg/mL *Eruca sativa* at 10.41%
- 0.04 µg/mL Vitamin D3.

The study was then developed in two different steps.

### STEP I

An absorption study was performed using the Transwell^{®} system, with human intestinal epithelial cells, CaCo-2: a widely used and validated model for studies on the absorption of orally administered compounds (approved by the FDA and the EMA).

The *in vitro* 3D model to test absorption and the mechanisms of transport through the intestinal barrier was used to conduct the following analyses:
- Analysis of cell viability through the MTT assay
- Analysis of reactive oxygen species (ROS) production
- Analysis of the Transepithelial Electrical Resistance (TEER) measurement
- Tight junction activity.

The cells seeded into the Transwell^{®} insert were maintained in a complete medium, changed every other day for 21 days before the stimulations in order to ensure the maturation and formation of intestinal microvilli. Maturation was complete when a transepithelial resistance (TEER) value ≥400/cm² was reached. The TEER was calculated using methods known to a person skilled in the art.

### STEP II

The product metabolised by the intestinal cells was placed directly in contact with a MC3T3-E1 and RAW 264.7 co-culture.

Damage to an *in vitro* model of osteoporosis induced by dexamethasone was simulated: In particular, dexamethasone 400 ng/mL for 3 days was used to induce the osteogenic damage.

At this point, the following were analysed on the *in vitro* induced osteoporosis model:
- Osteoblast (alkaline phosphatase) maturation mechanisms
- Bone recovery mechanisms (bone mineralisation)
- Calcification mechanisms
- Inhibition of osteoclast activity (TRAP activity)
- Reduction of bone resorption (formation of intraosseous voids).

### 2. Materials and methods

### MC3T3-E1 and RAW 264.7 cell cultures

A cell line of MC3T3-E1 osteoblasts purchased from ATCC was cultured in a Dulbecco's modified Eagle's medium (DMEM, Merck Life Science, Milan, Italy), integrated with 10% foetal bovine serum (FBS, Merck Life Science, Milan, Italy), 2 mM of L-glutamine (Merck Life Science, Milan, Italy) and 1% penicillin-streptomycin (Merck Life Science, Milan, Italy) at 37 °C and 5% CO2 [Park E, Kim J, Kim MC, et al. Anti-Osteoporotic Effects of Kukoamine B Isolated from Lycii Radicis Cortex Extract on Osteoblast and Osteoclast Cells and Ovariectomized Osteoporosis Model Mice. Int J Mol Sci. 2019;20(11):2784. Published 2019 Jun 6. doi:10.3390/ijms20112784]. The RAW 264. 7 monocytes purchased from ATCC were cultured in a Dulbecco's modified Eagle's medium (DMEM, Merck Life Science, Milan, Italy), integrated with 10% foetal bovine serum (FBS, Merck Life Science, Milan, Italy), 2 mM of L-glutamine (Merck Life Science, Milan, Italy) and 1% penicillin-streptomycin (Merck Life Science, Milan, Italy) at 37 °C and 5% CO2; the cells were differentiated into osteoclasts using RANKL + 10 ng/mL MCSF. The culture media were replenished every 2-3 days [Jarrar H, Çetin Altindal D, GÜmÜ 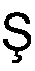 derello 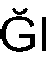 lu M. The inhibitory effect of melatonin on osteoclastogenesis of RAW 264.7 cells in low concentrations of RANKL and MCSF. Turk J Biol. 2020 Dec 14;44(6):427-436. doi: 10.3906/biy-2007-85. PMID: 33402869; PMCID: PMC7759193.]

### Alkaline phosphatase (ALP) activity test

After treatment, the cells were washed with PBS and lysed with the lysis buffer containing 50nM Tris-HCL (pH 7.4) and 1% Triton X-100 to collect the supernatants and determine the ALP activity and concentration of proteins. The ALP activity was determined with 4 mg/mL of 4-itrophenyl phosphate (4NPP) in 0.2 M of 2-amino-2-methyl-1-propanol with 4 mM of MgCl2 as substrate for 30 minutes at 37°C. The reaction was halted by 0.1 M NaOH, and the yellow solution was measured at 405 nm. The concentration of proteins was measuring using a Pierce BCA Protein Assay Kit (BCA, ThermoFisher Scientific, Waltham, MA, USA). The ALP activity was normalised at the concentration of the proteins and shown in terms of µmole/min/mg of protein normalises on the untreated control sample [Yodthong T, Kedjarune-Leggat U, Smythe C, Sukprasirt P, Aroonkesorn A, Wititsuwannakul R, Pitakpornpreecha T. Enhancing Activity of Pleurotus sajor-caju (Fr.) Sing β-1,3-Glucanoligosaccharide (Ps-GOS) on Proliferation, Differentiation, and Mineralization of MC3T3-E1 Cells through the Involvement of BMP-2/Runx2/MAPK/Wnt/β-Catenin Signaling Pathway. Biomolecules. 2020 Jan 27;10(2):190. doi: 10.3390/biom10020190. PMID: 32012654; PMCID: PMC7072289.].

### Alizarin red staining for mineralisation

In order to examine mineralisation, the MC3T3-E1 cells were placed in a differentiation medium at a concentration of 1 × 105 cells per well in 6-well plates. After 21 days, the medium was removed and stained with Alizarin red. The cells were washed three times with PBS and then fixed with ethyl alcohol at 75% for 30 minutes at 4°C. The cells were then washed with distilled water and stained with a 1% Alizarin red solution (pH 4.2) for 30 minutes at 37°C. The non-bonded dye was removed by washing it several times with distilled water. The mineralised nodules were observed using a low-magnification microscope and were photographed. Mineralisation of the matrix was quantified by adding 1mL of cetylpyridinium chloride 100 mM to each well and incubating it for 1 hour to dissolve and release the dye bonded to the calcium. Absorbance was measured at 570nm.

### Staining with tartrate-resistant acid phosphatase (TRAP) and TRAP activity assay

The mature osteoclasts were washed with DPBS and fixed in formaldehyde at 10% for 10 minutes. After being fixed, the cells were stained using a TRAP staining kit (Sigma-Aldrich Merck-life Sciences, Italy) according to the manufacturer's instructions. The cells were washed with deionized water and the TRAP-positive multinucleate cells (>3 nuclei) were counted on the inverted microscope (Olympus, Tokyo, Japan). To quantify the TRAP activity, 50µl of supernatant was mixed with 150µl of tartaric acid solution and after 30 minutes 50µl of NaOH 0.5 M was added, after which it was measured at 405 nm using an ELISA reader (Tekan). [Kim JH, Kim EY, Lee B, Min JH, Song DU, Lim JM, Eom JW, Yeom M, Jung HS, Sohn Y. The effects of Lycii Radicis Cortex on RANKL-induced osteoclast differentiation and activation in RAW 264.7 cells. Int J Mol Med. 2016 Mar;37(3):649-58. doi: 10.3892/ijmm.2016.2477. Epub 2016 Feb 1. PMID: 26848104; PMCID: PMC4771095.].

### Creation of a co-culture system for bone resorption

A new co-culture system was established using Transwell^{®} inserts (Corning Incorporated Number 3450, NY, USA). The differentiated RAW 264.7 cells were incorporated at a density of 5×105 cells/cm² in the lower compartment of each insert. The MC3T3-E1 cells were incubated at a density of 5×10⁴ cells/cm² in the upper compartment of the inserts. After each cell had been cultured for 24 hours, the Transwell inserts with the MC3T3-E1 cells were combined on the bone resorption assay plate with the RAW 264.7 cells. This co-culture system was maintained in DMEM supplemented with 10% foetal colt serum (FCS) with a penicillin-streptomycin-neomycin (PSN) antibiotic mixture (5 mg each of penicillin and streptomycin and 10 mg of neomycin/mL, GIBCO 15640, Life Technologies, Inc.) in 5% CO² at 37°C. After culturing for 5 days, the subsequent experiments were performed [Ishikawa S, Tamaki M, Ogawa Y, Kaneki K, Zhang M, Sunagawa M, Hisamitsu T. Inductive Effect of Palmatine on Apoptosis in RAW 264.7 Cells. Evid Based Complement Alternat Med. 2016;2016:7262054. doi: 10.1155/2016/7262054. Epub 2016 May 31. PMID: 27340419; PMCID: PMC4906184].

### OPG and RANKL activity

The OPG was analysed using the OPG/TNFRSF11B Duo Set (R&D Systems, Minneapolis, MN, USA), following the manufacturer's instructions [Galla R, Ruga S, Ferrari S, et. al. In vitro analysis of the effects of plant-derived chondroitin sulfate from intestinal barrier to chondrocytes. Journal of Functional Foods. 2022;105285, https://doi.org/10.1016/j.jff.2022.105285.]. Briefly, 100 µL of samples or standards were added to the wells and incubated for 2 hours at ambient temperature, protected against the light; after washing, 100µL of detection antibody was added to each well and incubated as previously described. After 2 hours, 100µL of the working dilution of Streptavidin-HRP A was added to each well and incubated for 20 minutes at ambient temperature. At the end of that time, 100µL of substrate solution was added to each well, incubated for 20 minutes at ambient temperature, and 50µL of stop solution to halt the enzymatic reaction. The optical density of each well was measured at 450 nm with a spectrometer (Infinite 200 Pro MPlex, Tecan). The results were interpolated with the standard curve (6.25-6.25 pg/ml) and expressed as a percentage (%) with respect to the control. The RANK was analysed using the Mouse RANKL ELISA Kit (abcam, Cambridge, UK), following the manufacturer's instructions [Wang W, Zhang LM, Guo C, Han JF. Resveratrol promotes osteoblastic differentiation in a rat model of postmenopausal osteoporosis by regulating autophagy. Nutr Metab (Lond). 2020;17:29. Published 2020 Apr 16. doi:10.1186/s12986-020-00449-9]. Briefly, 100 µL of samples or standards were added to the wells and incubated for 2.5 hours at ambient temperature, protected against the light; after washing them 4 times, 100µL of RANKL biotinylated detection antibody was added to each well and incubated for 1 hour at ambient temperature. After washing, 100µL of HRP-Streptavidin solution was added to each well and incubated for 45 minutes at ambient temperature. At the end of that time, 100µL of TMB One-Step Substrate Reagent was added to each well, incubated for 30 minutes at ambient temperature, and then 50µL of Stop Solution to halt the enzymatic reaction. The optical density of each well was measured at 450 nm with a spectrometer (Infinite 200 Pro MPlex, Tecan). The results were interpolated with the standard curve (from 2.74 to 2000 pg/ml) and expressed as a percentage (%) with respect to the control.

### 3. Results

### STEP I - Absorption study

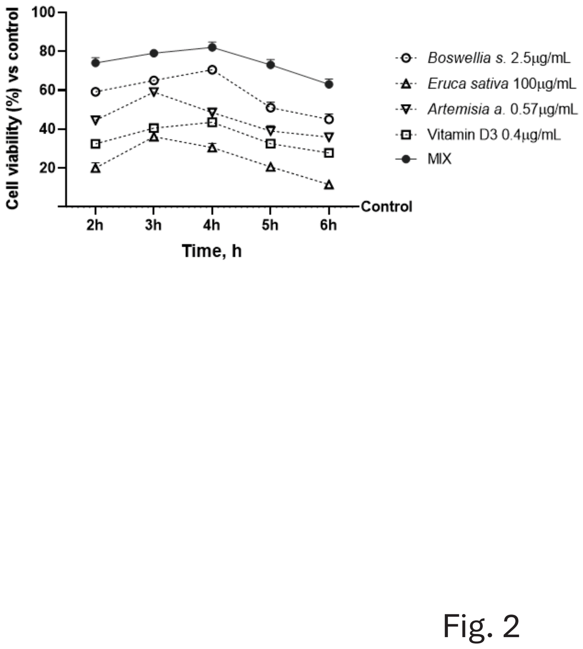

The analyses performed demonstrated that the tested substances are capable of stimulating cell viability without inducing cytotoxic effects; the data obtained show a cooperative effect of the MIX, as it induces an increase in viability compared to the individual substances (Figure 2).

### STEP I - Analysis of reactive oxygen species (ROS) production

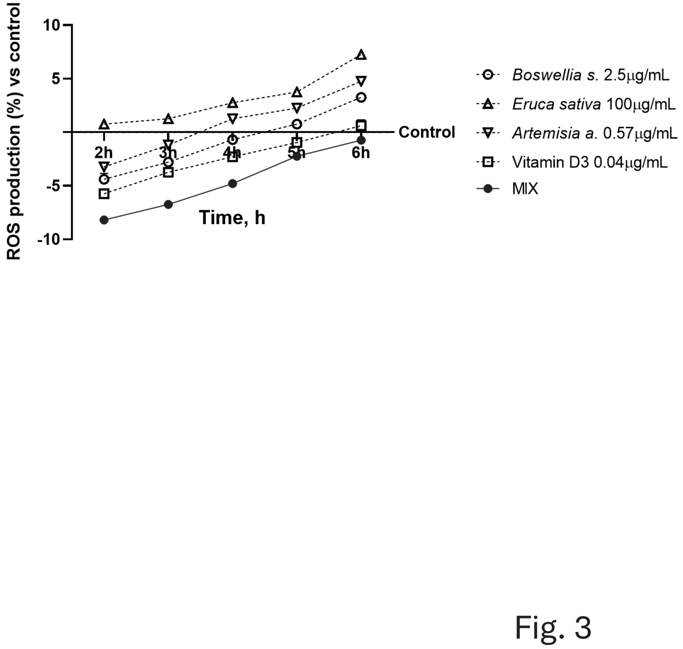

ROS production was inhibited/maintained within the physiological values of the individual components; in addition, the mixture had a protective effect, further promoting the antioxidant capacities of the substances analysed. ROS production following treatment with the MIX did, in fact, remain below the control values for the entire treatment period (Figure 3).

### STEP I - Analysis of the TEER measurement

Transepithelial resistance was measured to determine whether integrity of the intestinal tissue is maintained after treatment with the individual components and mixtures thereof.

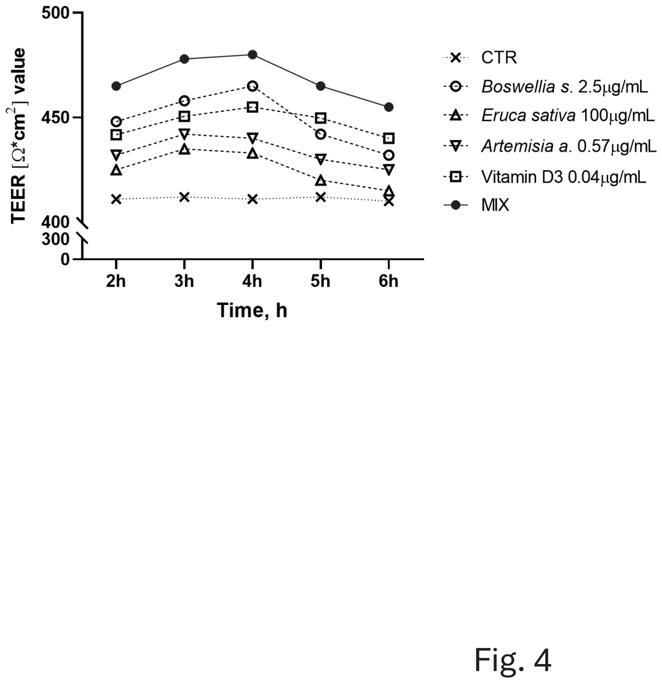

The safety of the molecules examined on the intestinal epithelium emerged from the analysis, confirming correct functioning of the ion exchanges (Figure 4).

### STEP I - Tight junction activity.

Tight junction analysis was conducted: in particular of Zo-1 (mediates adhesion); claudin (maintains structure) and occludin (contributes to stabilisation). This test is decisive for the barrier integrity and function (avoiding irritability or conditions that may lead to leaky gut syndrome). The Zo-1, claudin and occludin analysis confirmed the active role of the tight junctions in absorbing the compounds.

The analysis was conducted at 6h after the end the stimulation, to verify that there were no alterations in the intestinal structures.

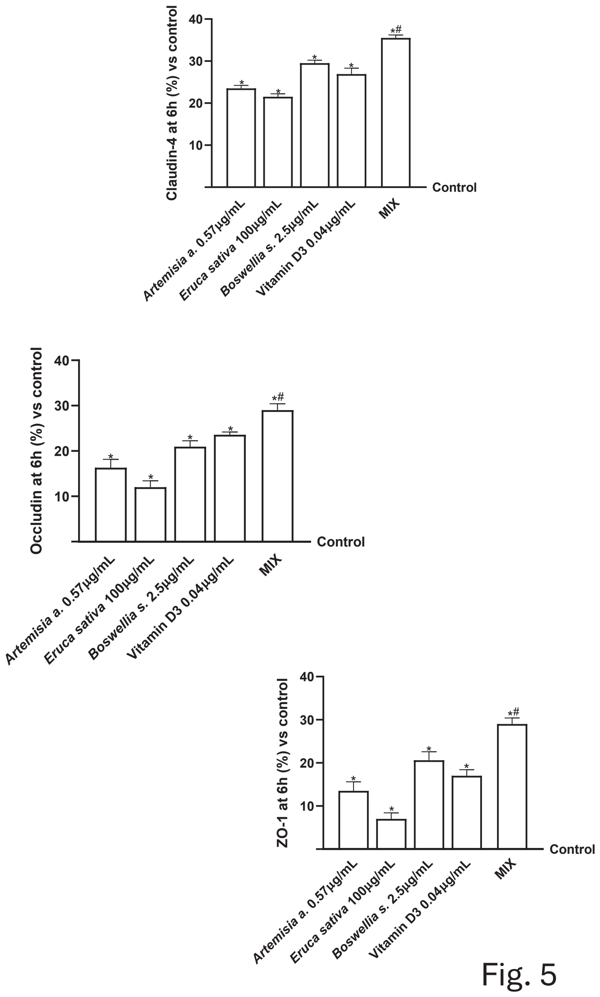

The data obtained confirm the correct functioning of intestinal integrity both in the presence of the individual compounds and, above all, in the presence of the MIX (p<0.05) (Figure 5). The MIX does, in fact, perform a significant activity on both the control and on the individual agents (p<0.05).

### STEP II - Osteoblast (alkaline phosphatase) maturation mechanisms

Alkaline phosphatase is an essential enzyme during the initial STEP of osteoblast differentiation. It is, in fact, considered to be an early osteogenetic differentiation marker, whereas mineralisation is the process observed during a subsequent STEP of osteoblast differentiation.

In order to confirm that the MC3T3-E1 cells differentiated into mature functioning osteoblasts, analysis of the activity of the alkaline phosphatase ALP (differentiation marker) and Alizarin red (mineralisation marker) was performed.

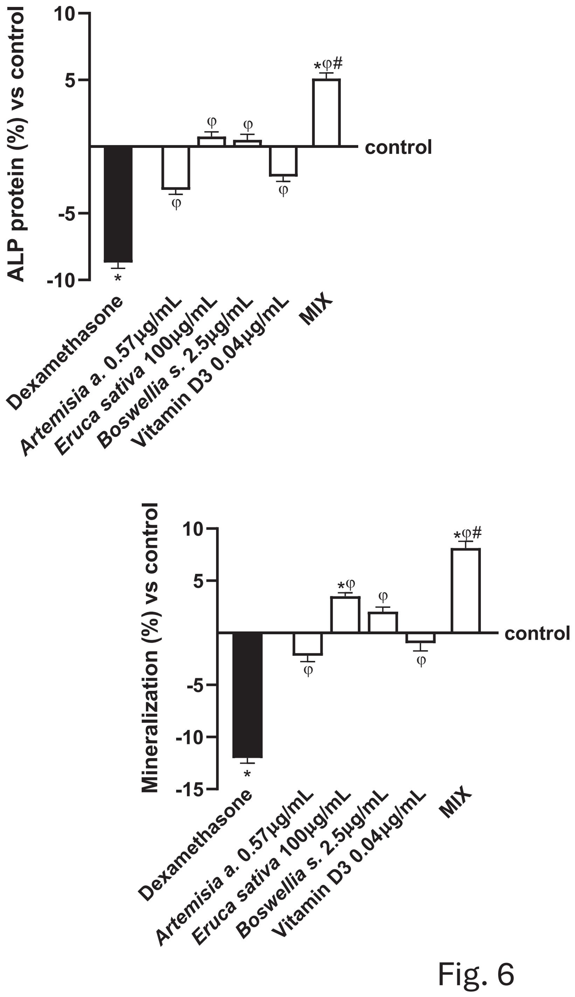

All the substances, on their own and combined, can increase the activity of the osteoblasts with respect to the damage induced with dexamethasone (p<0.05) (Figure 6).

In particular, the mixture (MIX) is capable of amplifying the beneficial effects on synthesis of the matrix and on mineralisation with respect to the damage, to the control and to the individual agents (p<0.05) (Figure 6). This demonstrates the capacity of the (MIX) to improve the functions of the osteoblasts.

STEP II - Analysis of bone mineralisation and calcification mechanisms Staining with Alizarin red was performed to measure the bone mineralisation induced by treatment of the MC3T3-E1 cells with the mixture.

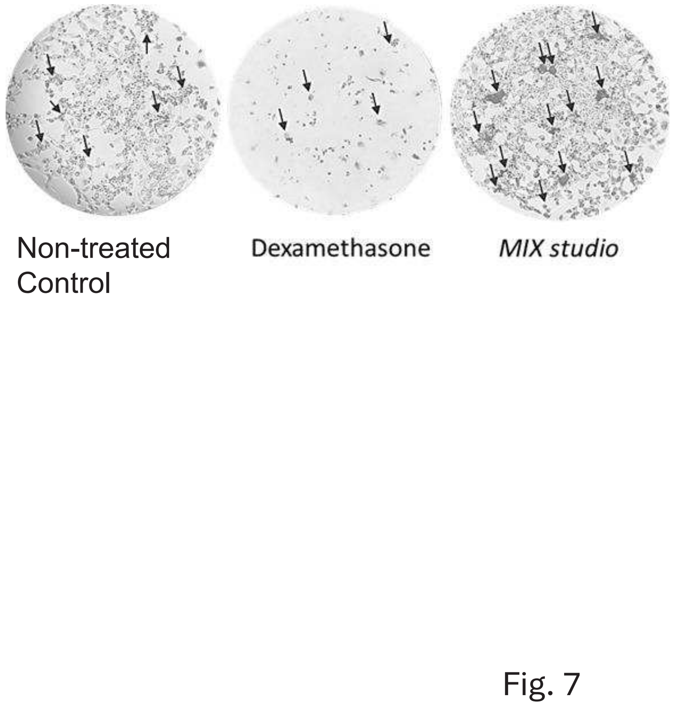

As can be observed from Figure 7, dexamethasone induces a major reduction in the depositing of calcium with respect to the control (p<0.05). In contrast, the MIX is capable of having beneficial effects on the depositing of calcium and consequently on mineralisation with respect to the damage and to the control (p<0.05).

The number of calcium deposits detected is indicated on Table 7 and in Figure 7, where the calcium deposits are indicated by the arrows. The higher number of arrows of the MIX in Figure 7 represents the higher number of calcium deposits.

**Table 7**

| **Sample** | **Mean ± SD** |
|---|---|
| Untreated control | 54,68 ± 1.53 |
| Dexamethasone | 5,31 ± 1.37 |
| MIX | 82,74 ± 2.11 |

### STEP II - Analysis of inhibition of osteoclast activity (TRAP activity)

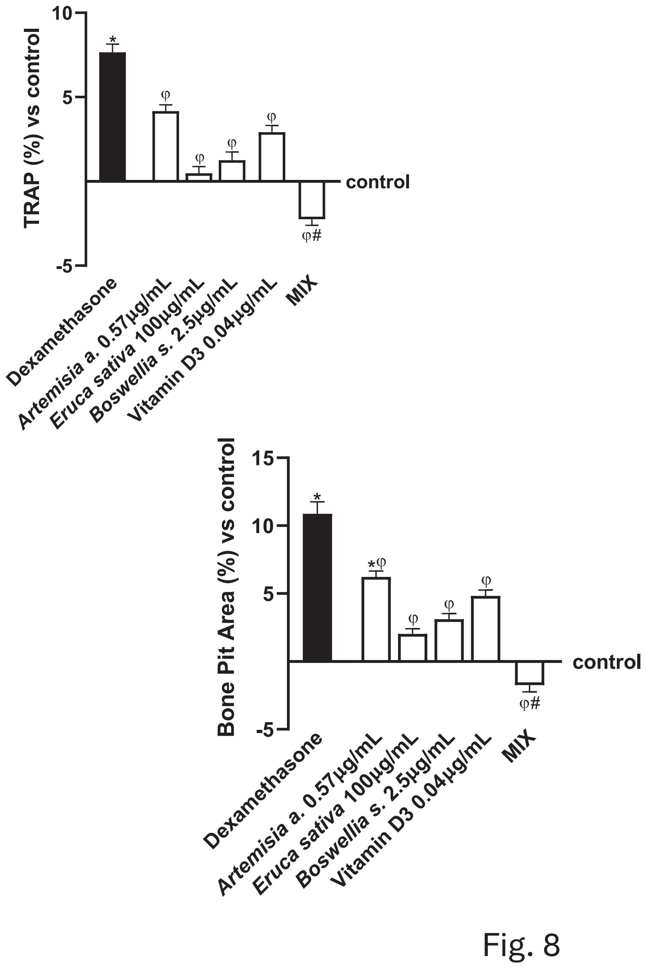

Differentiation of the RAW 264.7 cells in osteoclasts was examined through TRAP analysis in a 3D co-culture with MC3T3-E1 cells. The individual agents and the MIX were capable of reducing the TRAP activity with respect to the damage induced (p<0.05) (Figure 8, left-hand panel).

This data confirms the efficacy of the mixture in inhibiting osteoclast activity.

### STEP II- Reduction of bone resorption (formation of intraosseous voids)

In addition, further experiments were conducted to check the role on the osteoclast resorption activity. The bone resorption capacity of the RAW 264.7 cells was examined using a kit for dosing bone resorption in the 3D co-culture of RAW 264.7 and MC3T3-E1 cells.

The bone resorption activity was measured by measuring the intensity of the fluorescence in the medium with stimulation. The intensity decreased significantly with all the substances, on their own and combined, improving the activity of the osteoblasts and reducing the function of the osteoclasts with respect to the damage (p<0.05). In particular, the mixture was capable of reducing the activity of the osteoclasts with respect to the damage, to the control and to the individual agents (p<0.05), confirming the capacity of the MIX to inhibit osteoclast activity, suppressing osteoclastogenesis and bone resorption (Figure 8, right-hand panel).

### STEP II - Analysis of reduction of bone resorption - RANKL/OPG

The data obtained suggest that the MIX performs a crucial role in mineralisation of the newly formed bone, increasing it during differentiation of the osteoblasts.

It is known that osteoclast and osteoblast cells communicate with each other through RANKL/OPG: osteoclastogenesis is, in fact, controlled by RANKL in a dose-dependent way, whereas OPG reverses the effects of RANKL by acting as a decoy receptor. In this manner, the bone resorption activity is reduced. The protein levels were measured on the RAW 264.7 co-culture with MC3T3-E1 cells. From the results obtained, it can be observed how the homoeostasis of bone remodelling is maintained by the balance between bone resorption and formation after treatment even during an osteoporotic condition, leading to the hypothesis that the MIX can slow down the onset of bone diseases such as osteoporosis. Specifically, the MIX has a significant activity not only with respect to the damage, but also to the control and to the individual agents (p<0.05) (Figure 9).

### 4. Conclusions

The data obtained represent important experimental evidence and confirm, advantageously, that the mixture comprising extracts of *Artemisia annua, Boswellia serrata, Eruca sativa Mill.* and vitamin D3:
- crosses the intestinal epithelium without altering the integrity thereof
- improves the functions of the osteoblasts
- plays a crucial role in mineralisation of the newly formed bone, increasing it during osteoblast differentiation
- plays a crucial role in calcification of the newly formed bone
- inhibits osteoclast activity, reducing osteoclastogenesis and bone resorption
- maintains homoeostasis of bone remodelling by preserving the balance between resorption and formation even during an osteoporotic condition.

## Claims

1. A mixture comprising or, alternatively, consisting of:
i) an *Artemisia* extract;
ii) a *Boswellia* extract;
iii) an *Eruca* extract; and
iv) at least one vitamin D.

2. The mixture according to claim 1, wherein said *Artemisia* extract is an *Artemisia annua* extract, and/or said *Boswellia* extract is a *Boswellia serrata* extract, and/or said *Eruca* extract is an *Eruca sativa Mill.* extract.

3. The mixture according to claim 1 or 2, where the *Artemisia* extract comprises artemisinin in a concentration comprised from 80% to 100% by weight; more preferably, comprised from 85% to 99.9% by weight; even more preferably, comprised from 90% to 99.7% by weight, with respect to the total weight of the extract.

4. The mixture according to any one of the preceding claims, where the *Boswellia* extract comprises at least one keto-boswellic acid, preferably said at least one keto-boswellic acid is 3-acetyl-11-keto-β-boswellic acid (AKBA).

5. The mixture according to any one of the preceding claims, wherein said at least one keto-boswellic acid is present in said *Boswellia* extract in a concentration comprised from 20% to 70% by weight; more preferably, comprised from 25% to 60%; even more preferably, comprised from 30% to 50% by weight, with respect to the total weight of the extract.

6. The mixture according to any one of the preceding claims, wherein the *Eruca* extract comprises icariins, preferably in a concentration comprised from 5% to 50% by weight, preferably, comprised from 10% to 40% by weight; more preferably, comprised from 15% to 25% by weight, with respect to the total weight of the extract.

7. The mixture according to any one of the preceding claims, wherein said at least one vitamin D is chosen from the group comprising, or alternatively consisting of, vitamin D1, vitamin D2, vitamin D3, vitamin D4,and vitamin D5, or mixtures thereof; preferably, said vitamin D is chosen from among vitamin D2 and vitamin D3, or mixtures thereof; more preferably, said vitamin D is vitamin D3.

8. A composition comprising the mixture according to any one of claims 1 to 7 and, optionally, at least one acceptable pharmaceutical or food grade additive and/or excipient.

9. The composition according to claim 8, where the Artemisia extract is present in a concentration comprised between 10 and 25% weight/weight of the composition, preferably comprised between 12 and 18% w/w.

10. The composition according to claim 8 or 9, where the Boswellia extract is present in a concentration comprised between 30 and 65% weight/weight of the composition, preferably comprised between 45 and 55% w/w.

11. The composition according to any one of claims 8 to 10, where the Eruca extract is present in a concentration comprised between 20 and 45% weight/weight of the composition, preferably comprised between 25 and 40%.

12. The composition according to any one of claims 8 to 11, where the at least one vitamin D is present in a concentration comprised between 0.005 and 0.007% weight/weight of the composition, preferably comprised between 0.006 and 0.008 % w/w.

13. The mixture according to any one of claims 1 to 7 or the composition according to any one of claims 8 to 12, for use as a medicament.

14. The mixture according to any one of claims 1 to 7 or the composition according to any one of claims 8 to 12, for use in the prevention and/or slowdown and/or in treatment of osteoporosis.

15. A non-medical nutraceutical use of the mixture according to any one of claims 1 to 7 or the composition according to any one of claims 8 to 12 to maintain bone homoeostasis, and/or to maintain bone mineral density and/or to maintain bone microarchitecture and/or for the reduction of the risk of fractures caused by bone fragility in an individual.
